Europäisches Patentamt

European Patent Office

Office européen des brevets

(19)

(11) Publication number: **0 393 045 B1**

# EUROPEAN PATENT SPECIFICATION

(12)

(45) Date of publication of patent specification: **29.03.95**  (51) Int. Cl.⁶: **C12N  15/70**, C12P 21/00

(21) Application number: **88907720.2**

(22) Date of filing: **09.09.88**

(86) International application number:
**PCT/GB88/00747**

(87) International publication number:
**WO 89/02465 (23.03.89 89/07)**

(54) **Fv antibody fragment production.**

(30) Priority: **09.09.87 GB 8721230**
        **23.11.87 GB 8727431**

(43) Date of publication of application:
**24.10.90 Bulletin  90/43**

(45) Publication of the grant of the patent:
**29.03.95 Bulletin  95/13**

(84) Designated Contracting States:
**AT BE CH DE FR GB IT LI LU NL SE**

(56) References cited:
**EP-A- 0 088 994**
**EP-A- 0 121 386**
**EP-A- 0 125 023**
**EP-A- 0 216 747**
**WO-A-85/03949**

(73) Proprietor: **CELLTECH LIMITED**
**216 Bath Road**
**Slough**
**Berkshire SL1 4EN (GB)**

(72) Inventor: **FIELD, Helen**
**68 Banbury Road**
**Oxford**
**Oxfordshire OX2 6JP (GB)**
Inventor: **REES, Anthony, Robert**
**17 Narrow Hill**
**Witney**
**Oxfordshire (GB)**
Inventor: **YARRANTON, Geoffrey, Thomas**
**8 Sherwood Road**
**Winnersh, Nr. Wokingham**
**Berkshire RG11 7NJ (GB)**

(74) Representative: **Mercer, Christopher Paul et al**
**Carpmaels & Ransford**
**43, Bloomsbury Square**
**London WC1A 2RA (GB)**

## Description

Field of the invention

This invention relates to recombinant DNA technology and in particular to processes for the production of polypeptides, especially immunologically functional immunoglobulin fragments such as immunoglobulin Fv fragments and derivatives thereof.

Background to the Invention

Over recent years recombinant DNA technology has been applied to the production of a wide range of useful protein and polypeptide products. Thus the techniques of recombinant DNA genetic manipulation allow for the transformation of a host organism with a foreign gene. The transformed host organism cells may then produce the protein or polypeptide for which the foreign gene codes. Such modified, or transformed, host cells provide a reproducible culture source for the large scale production of polypeptides or protein using industrial fermentation techniques.

Many of the products to which recombinant DNA techniques are applied are polypeptides such as human or animal hormones. It has been found that when the polypeptide is of relatively small size, e.g. of less than about 150 amino acids in length, only low concentrations of the polypeptide product accumulate in the host cells when the product is produced as a direct expression product, e.g. as a methionine polypeptide product. Such low accumulations of product, coupled with the necessary time consuming and expensive processes of purification, render commercial working uneconomic. It appears that this low accumulation of product may be due, at least in part, to proteolytic turnover of the foreign product by the host cells.

In order to increase the yield of foreign products, such products have been produced as fusion proteins in which the polypeptide product is fused to a larger protein which is known to accumulate in the host organism. This may be achieved by ligating a gene coding for a protein known to be produced abundantly in the selected host organism, with a gene coding for the desired product, in the correct reading frame and without intervening stop codons. Examples of such abundantly produced proteins are anthranylate synthetase (Trp gene product), β-galactosidase (lacZ gene product), and chloramphenicol acetyltransferase (CAT). Such fusion proteins must be cleaved to recover the desired polypeptide. In general, the procedures used for recovery and purification of such fusion proteins and recovery of the desired products from the fusion proteins are complex and costly. Furthermore, the proteins referred to above, which are used to form a fusion protein with desired polypeptides, are large in comparison with a typical polypeptide product. The desired polypeptide therefore represents only a small percentage of the fusion protein produced by the transformed host cells, and thus the efficiency of the production process is low.

The limitations to the use of recombinant DNA technology, with regard to the production of relatively small size polypeptides, has been particularly felt in the application of recombinant DNA technology to the production of immunologically functional immunoglobulin fragments and in particular immunoglobulin Fv fragments.

For the purpose of the present description the term "immunologically functional immunoglobulin fragment" means an immunoglobulin fragment which has specific binding affinity for a corresponding antigenic determinant.

In recent years recombinant DNA technology has been applied to the preparation of immunoglobulin (Ig) products, i.e. antibodies and parts thereof. Thus, DNA sequences coding for complete heavy and light Ig chains have been expressed in bacteria and the complete heavy and light chains reconstituted in vitro to give immunologically functional molecules (Boss et al., Nucleic Acids Research (1984), volume 12 No. 9, pages 3791-3806 and Cabilly et al., Proc. Natl. Acad. Sci. USA (1984), volume 81 pages 3273-3277).

The application of recombinant DNA technology to Ig production has opened up the possibility of producing engineered Igs or Ig fragments. For example, "chimeric antibodies" have been prepared (published European Patent Application EP-A-0171496 - Research Development Corporation Japan; published European Patent Application EP-A- 0173494 - Stanford University, and published International Patent Application WO 86/01533 - Celltech) comprising mouse variable region and human constant region amino acid sequences. Also "altered antibodies" have been produced (International Patent Application WO 86/01533 - Celltech) in which constant region domains have been replaced with a polypeptide of different function, such as an enzyme. Furthermore, "humanised" antibodies have been prepared (published European Patent Application EP-A-0239400 - Winter) in which the hypervariable/complementarity determining regions of a mouse monoclonal antibody have been grafted into the variable framework regions of a human antibody.

Furthermore, it has been proposed to prepare immunologically functional Ig fragments including Fab', F (ab')$_2$ (published European Patent Application EP-A-0125023 - Genentech) and Fv (published European Patent Application EP-A-0008994 - Schering) fragments by recombinant DNA technology. Such antibody fragments have potential therapeutic uses: they lack effector functions, are less immunogenic, more soluble and potentially more susceptible to transfer across cell membranes than whole antibody molecules. Also they may be used to prepare altered antibody molecules in which the fragments are prepared, fused to other functional polypeptides, e.g. enzymes, toxins etc., for instance, at the gene level by expression of fused DNA sequences.

Moreover, ease of production of antibody fragments permits various scientific investigations: for example, a range of mutant antibody fragments may be prepared to study and analyse the relationship between primary structure and binding site specificity. Also antibody fragments may be used for structural studies of antibodies: Fvs may be easier to crystallise than whole antibody molecules and thus permit structure determination by X-ray crystallography. Also NMR assignments are simpler with small antibody fragments.

Published European Patent Application EP-A-0008994 (Schering Corporation) proposes the use of recombinant DNA technology for production of immunologically functional Fvs. This patent application describes the preparation of a double stranded DNA sequence which codes for a variable region of a light or heavy chain of an Ig specific for a predetermined ligand but lacking nucleotides coding for amino acid residues superfluous to said variable region. The expression of such DNA sequences in E. coli host cell is described and it is suggested that Fv heavy and light chain products are isolated from the supernatant obtained after centrifugation of lysed host cells. However, no results of product yield or reconstitution of immunologically functional Fvs are given

Hitherto, there have been no reports in the literature describing the successful production of immunologically functional Fvs using recombinant DNA techniques involving expression of light and heavy chain variable polypeptides in bacteria. It appears therefore that, to date, a satisfactory solution to the problem of producing immunologically functional Fvs in bacteria, has not been forthcoming.

It has been found, unexpecdly, that it is possible to accumulate satisfactory levels of relatively small polypeptide products, such as immunologically functional Fvs, by direct expression of genes coding for the polypeptide products in bacterial host cells.

Summary of the Invention

Accordingly, in a first aspect, the present invention provides a process for the production of an immunologically functional Fv antibody fragment comprising direct expression of heavy and light chain polypeptides comprising only complementary variable domains in a bacterial host strain which is protease-deficient.

The Fv fragments may comprise natural antibody fragments, altered antibody fragments (i.e. variable Ig domains plus an additional polypeptide sequence having different, non Ig function e.g. an enzyme or toxin), "humanised" antibody fragments (i.e. wherein the Ig sequence comprises hypervariable/complementarity determining regions from one species, e.g. a mouse, including polypeptide fragments of cell surface receptors. In particular the invention may be applied to the preparation of antibody fragments, especially immunologically functional antibody fragments.

Accordingly, in a second aspect, the present invention provides a process for the production of an immunologically functional antibody fragment comprising expression and preferably reconstitution of heavy and light chain polypeptides comprising complementary variable domains characterised in that an inducible expression system is used in combination with a protease deficient bacterial host strain.

The process of the invention may be applied to production of antibody fragments in general, provided the fragment has binding specificity for a corresponding antigenic determinant. It will be appreciated, also, that the heavy and light chain polypeptides are preferably of sufficient heterophilicity to permit association of heterodimer molecules. Thus the process may be used for production of (Fab')$_2$ and Fab' antibody fragments. Preferably, the antibody fragment is an Fv fragment.

The antibody fragments may comprise natural antibody fragments, chimeric antibody fragments, (i.e. variable domains derived from one species and class of antibody and remaining Ig sequences derived from another species or class of Ig), altered antibody fragments (i.e. variable Ig domains plus an additional polypeptide sequence having different, non Ig function e.g. an enzyme or toxin), "humanised" antibody fragments (i.e. wherein the Ig sequence comprises hypervariable/complementarity determining regions from one species, e.g. a mouse, grafted within framework regions of another species e.g. human), and "engineered" antibody fragments (i.e. wherein the Ig amino acid sequence has been altered from the natural sequence, e.g. by site directed techniques, with a view to altering a characteristic of the molecule e.g. antigen binding affinity or specificity - for instance, along the lines described by Roberts et al., Nature (1987). The antibody fragments may comprise suitable combinations of the above types of antibody fragment. Typically, the antibody fragments comprise substantially complete heavy and light chain variable domains which may be derived from a single or more than one species.

The antibody fragments may have any desired antigen specificity. For example, the antibody fragments may have specificity for a cell specific antigen e.g. a tumour marker, T cell marker, etc. Alternatively, the fragment may have specificity for compounds of therapeutic or analytical interest, e.g. fibrin, or hormones etc. In particularly preferred embodiments, the antibody fragments comprise Fv/enzyme fragments, e.g. anti-fibrin Fv/fibrinolytic enzyme fragments, or anti tumour Fv/enzyme fragments. The specificity of the antibody fragment may be the specificity of a natural antibody, humanised antibody or engineered antibody.

The inducible expression systems used in the process of the invention are systems in which vector copy number, and preferably also expression of DNA coding for the desiredpolypeptide or polypeptides, is inducible by external influences, such as temperature or metabolite. For example, the expression system may comprise a runaway replication vector of the general type described in British Patent Specification GB-B-1557774 (Alfred Benzon A/S).

Preferably, however, the expression system comprises a dual origin vector, of the type described in British patent specification No. GB-A-2136814. A dual origin vector is a vector comprising two replication systems; a first origin of replication resulting in a low copy number and stable inheritance of the vector, and a second, high copy number, origin of replication at which replication is directly controllable as the result of replacement or alteration by DNA manipulation of the natural vector sequence(s) which control replication at said second origin.

Accordingly, in a second aspect, the invention provides a copy number inducible bacterial expression vector for the production of an immunologically functional Fv antibody fragment in a protease-deficient bacterial strain containing a DNA sequence coding for a polypeptide comprising a variable domain only wherein the DNA sequence is located within the vector such that it is expressed as a direct expression product.

Expression of the polypeptides may be optimised by suitable vector adjustment. The ribosome binding site length and sequence around the AUG start codon, may be optimised for polypeptide expression. Also the Shine Dalgarno-ATG (SD-ATG) distance may be adjusted to optimise expression levels. For example, we have found that a relatively long ribosome binding site sequence (nine nucleotides) gave enhanced levels of expression of heavy chain variable polypeptide. Also we have found that SD-ATG distances of greater than 5, preferably 10-14, nucleotides in length are desirable.

The protease deficient bacterial host strains used in the process of the invention are typically constitutively protease deficient and in particular include constitutively protease deficient strains of E. coli. We have found that E. coli B host cells are not satisfactory for use in the process of the invention. With E. coli B, unsatisfactory levels of polypeptide product accumulate in the host cells. It was a surprising finding that other protease deficient strains permit accumulation of satisfactory levels of polypeptide product.

It may be noted that E. coli B strains are "naturally" lon- and as such they do not appear to be suitable as host strains for polypeptide production. Recent investigations have indicated that the lon gene is present in E. coli B strains and may be expressed under certain conditions such as heat stress. Thus E. coli B strains may not be truly protease deficient but retain some protease activity.

The protease deficient strain used in this invention may comprise a lon- strain or preferably an htpR- strain, e.g. E. coli CAG 456. More preferably a multiply protease deficient strain is used, e.g. a doubly deficient strain, including especially strains which are both htpR- and lon-, such as E. coli CAG 629 or similar strains.

Accordingly, in a third aspect, the invention provides protease deficient bacterial host cells transformed with a vector according to the third aspect of the invention.

Following expression, the polypeptide product may be recovered and further treated as desired. Following expression, the heavy and light chain fragments may be recovered and reconstituted in vitro using methods similar to or the same as those known in the art (e.g. Boss et al., loc. cit; Cabilly et al., loc cit). We have found that the polypeptide products typically accumulate within the host cells in the form of insoluble inclusion bodies. The polypeptide products may be recovered as part of the insoluble fraction after cell lysis. Any suitable method including Guanidine HCl or lysozyme treatment or French Press may be used to lyse the cells. After recovery, the insoluble polypeptide products may be solubilised, denatured and renatured using techniques well known in the art including treatment with chaotropic agents followed by dialysis and/or treatment with alkali followed by neutralisation, e.g. as described in British patent specification GB-B-2138004.

In accordance with the present invention we have found that the combination of inducible expression system and protease deficient bacterial host cells leads to accumulation of sufficient levels of light and heavy chain polypeptides to permit reconstitution of immunologically functional Fv fragments.

The invention is further described by way of illustration only in the following examples, which refer to the accompanying diagrams. The examples describe the production of recombinant heavy and light chain immunoglobulin variable region polypeptide fragments. It will be appreciated, however, that the invention is widely applicable to the production of relatively small polypeptides in general.

Brief Description of the Diagrams

This invention is further described, by way of illustration only, in the following examples, which refer to the diagrams, in which,

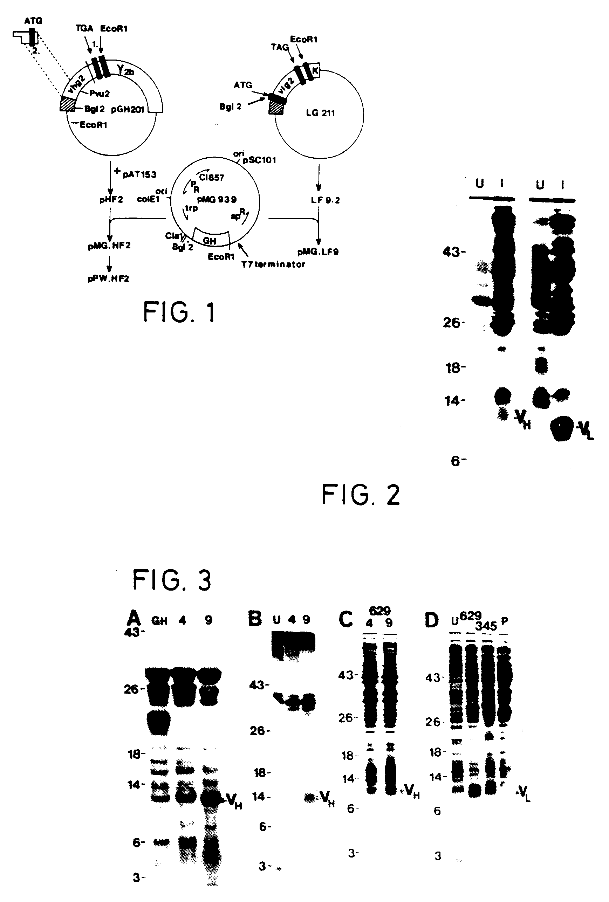

Figure 1 illustrates construction of rFv genes from full length Gloop2 clones; mutations are shown by black boxes in the double-stranded DNA. Hatched areas represent the signal sequences. vhg2: the heavy-chain was mutated at the 3′ end of the variable region (1) making $2+2$ base changes using an oligonucleotide 31 bases long and creating a TGA stop codon and a 3′ EcoR1 site. The EcoR1 fragment including vhg2 was excised and subcloned into pAT153. The existing Bg12-Pvu2 fragment was replaced with a linker (2) restoring the coding sequence and restriction sites, and containing an ATG start codon, encoding formyl-Met in E. coli, vlg2: double SDM using a 35mer and a 33mer at the 5′ and 3′ ends of the variable coding sequence, respectively made 8 and $3+4$ base changes. The growth hormone GH, was excised from pMG939 via its 5′ Bg12 and 3′ EcoR1 sites, and replaced by either the vhg2 or vlg2 Bg12-EcoR1 fragments, to give pMG.HF2 or pMG.LF9, respectively. pPW.HF2 contains the 9 bp SD but is otherwise identical to pMG.HF2;

Figure 2 illustrates expression of the rFv genes in E. coli; detection of $V_H$ and $V_L$ by biosynthetic labelling in cells without protease-deficiences (1B373). Autoradiographed SDS-PAGE (15%) of either uninduced (U) or induced (I) cells labelled for 2 minutes. The degradiation of $V_L$ is apparent even after 2 minutes;

Figure 3 illustrates augmenting the yield of $V_H$ with a 9-bp Shine-Dalgarno sequence 9SD); (A) Autoradiogram of 15% SDS-PAGE of in vitro translation products from pMG939 9GH0, pMG.HF2 with 4 bp SD (4), and pPW.HF2 with 9 bp sd (9): no $V_H$ products was produced in 30 minutes from pMG.HF2 (B) in vivo, Western blotting analysis of whole cells electrophoresed on 15% SDS-PA gels: pMG.HF2 (4) and pPW.HF2 (9) produced in CAG529 4.5 hours after induction. (C) Coomassie-blue stained 15% SDS-PAGE OF 1 $A_{600nm}$ samples 6 hours after induction of pMG.HF2 (4) and pPW.HF2 (9) in CAG629. (D) Coomassie-blue stained 15% SDS-PAGE of 1 $A_{500nm}$ cell extracts transformed with pMG.LF9: U = time 0 after induction; at time 6 hours after induction: 629 = in host strain CAG629; 456 = in host strain CAG456; P = htpR$^+$ parent strain, SC122. $V_L$ accumulated in CAG456 to half the level found in CAG629;

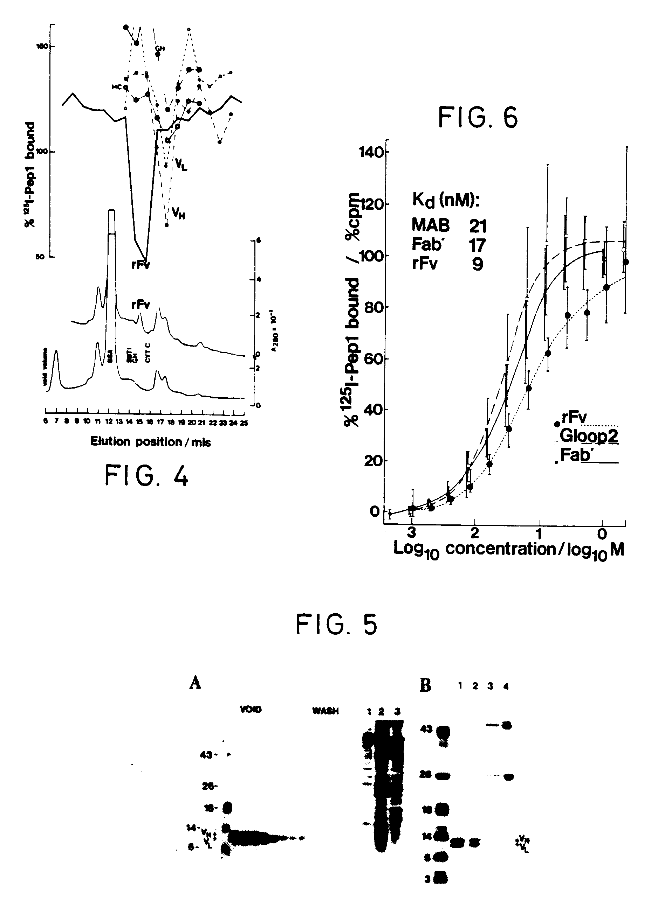

Figure 4 illustrates demonstration of a heterodimeric rFv complex; the $A_{280nm}$ elution profiles (below) from Superose 12 of the rFv-containing reconstitutions (upper) and host cell reconstitutions (lower) are compared with the Pep-1 binding activities obtained for each fraction (top), for rFv and controls: $V_H$ or $V_L$ reconstituted separately, growth hormone (GH) and host cells (GH). Protein concentrations are within a factor of 2, with respect to $V_H$ and $V_L$. Compare the elution positions of markers: bovine serum albuin (BSA) 66 kDa, growth hormone (GH) 22.5 kDa, soybean trypsin inhibitor (SBTI) 20.1 kDa and cytochrome c (CYT C) 12.4 kDa. Most of the monomer activity resided in reconstituted $V_H$;

Figure 5 illustrates the final step in the purification of rFv (Gloop2); (A) silver-stained 15% SDS-PAGE of fractions eluting from DEAE at pH 6.34 (20mM Tris), shoeing rFv eluting with high purity in the void volume (VOID), and more slowly during subsequent washing (WASH). $V_H$ appeared light yellow on silver staining, whereas $V_L$ was dark. Fractions were eluted with a 0-0.2M sodium chloride gradient and pooled (represented in lanes 1,2 and 3). (B) Coomassie-blue stained 15% SDS-PAGE of purified rFv fractions (lanes 1,2, where 2 is a mixture of rFv from washes and fractions eluted from DEAE at $\simeq$ 70mM NaCl in 15mm Tris pH 7.5). Lanes 3 and 4 show 2 $\mu$g and 5 $\mu$g whole Gloop2, separated into its constituent heavy and light chains. rFv was likewise separated into its constituents, $V_H$ and $V_L$ migrating with apparent $M_r$ of 11.5 and 9 kDa, respectively;

Figure 6 illustrates purified rFv and its activity compared with Gloop2 and Fab′ (Gloop2); each point is a mean of 8 data points, comprising 2 quadruplet determinations. Bars indicate the highest and lowest values obtained. $K_d$s are obtained by a computer program using parametric and non-parametric analyses. The $K_d$ values shown are a mean of these figures. Murine IgG (Sigma) controls showed no Pep1 binding: values were $\simeq$ 100% at all concentrations (not shown);

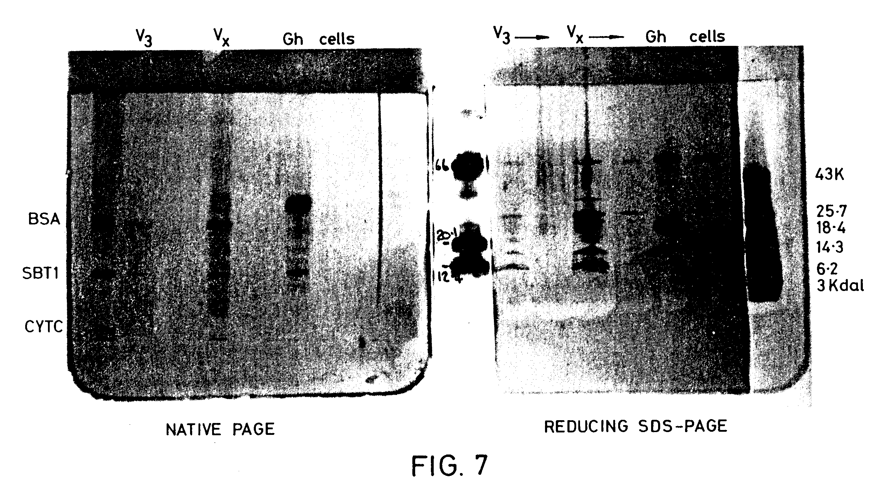

Figure 7 illustrates the fractionated Fv (Gloop2) reconstitutions; fractions 14 and 15 from Superose 12 fractionated Fv(Gloop2) reconstitutions, which fractions exhibit Pep1 binding activity, were electrophoresed on a Phast gel system (Pharmacia). Fractions 14 and 15 from Fv prepared with ($V_x$) and without ($V_3$) lysozyme were compared with control fractions, 14 and 15, from fractionated reconstitutions of cells containing growth hormone (GH) or cells, using both native and reducing SDS-PAGE. A band representing Gloop2 Fv was clearly present on native PAGE. It migrated at high molecular weight, consistent with it being a dimeric complex with a size close to but smaller than that of growth hormone (at 22.5kDa). Reducing SDS-PAGE indicated the presence of $V_H$ and $V_L$ in these Pep1 binding fractions, as a characteristic doublet migrating at a position consistent with a molecular weight of around 14 kDa;

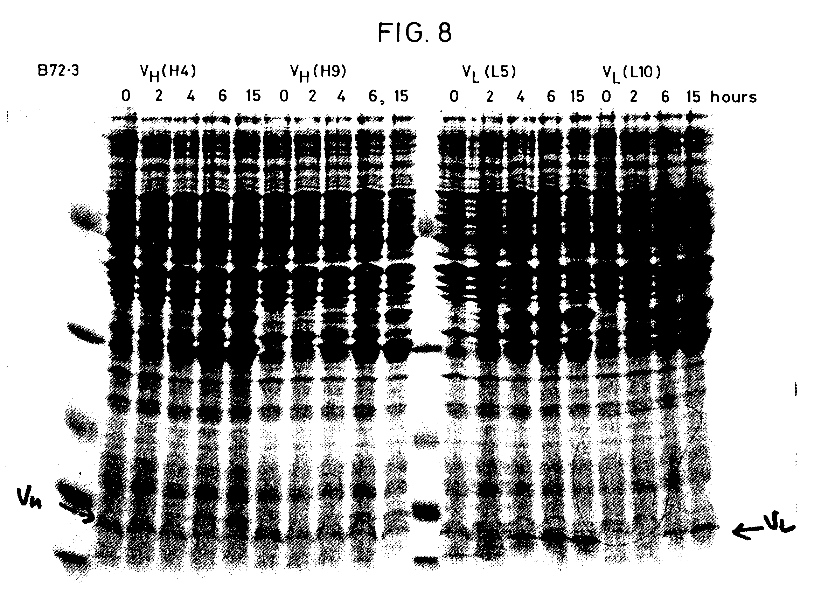

Figure 8 illustrates production of both heavy and light polypeptide chains; Coomassie-blue stained 15% SDS-PAGE of whole cells, sampled at various times after induction (at time = 0). Two identical constructs for B72.3 $V_H$ and for $V_L$ (pMG.ROH4, pMG.ROH9, pMG.ROL5 and pMG.ROL10) were transformed into host strain E. coli CAG629 and induced for protein synthesis. Times of sampling were, in order, 0, 2, 4, 6 and 15 hours (for the last time course the 4 hour sample was omitted). $V_H$ and $V_L$ both accumulated to a maximum level after 6 hours. Approximately equimolar amounts were synthesised, intermediate between levels obtained from Gloop2 $V_H$ and $V_L$;

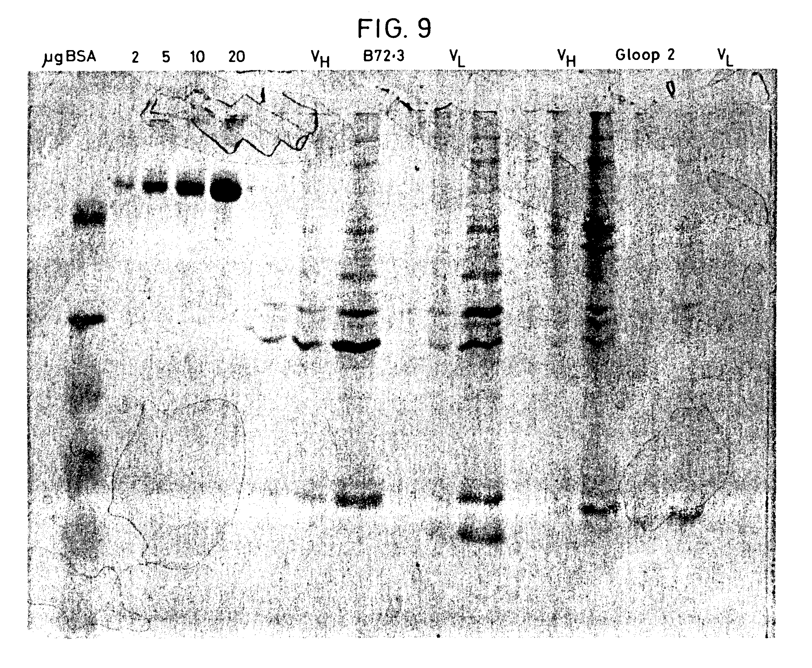

Figure 9 illustrates the appearance of the $V_H$ and $V_L$ polypeptides in the insoluble fraction of host cells; insoluble fractions of cells were washed stringently with 2% Triton X100 (buffered) and solubilised in 7.6M guanidine buffer (see Cabilly et al., 1984). These contain the polypeptides $V_H$ and $V_L$, as assessed by Coomassie-blue stained 15% SDS-PAGE of different loadings of B72.3 $V_H$, $V_L$ and Gloop2 $V_H$ and $V_L$, in order. Lysozyme migrated at a marginally higher position than B72.3 $C_H$ in the B72.3 examples; and

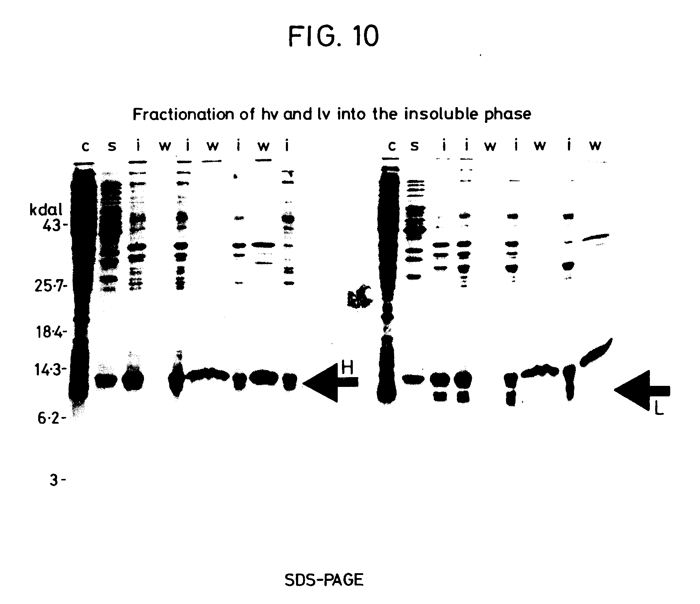

Figure 10 shows Coomassie-blue stained SDS-PAGE (15%) showing recombinant Gloop2 $V_H$ and $V_L$ expressed as insoluble material in Escherichia coli; c = $A_{600}$ cells, i = insoluble material, s = soluble fraction, w = supernatants from pellet (i) washes. H = $V_H$ and L = $V_L$.

## Detailed Description of the Embodiments

### Example 1

The Fv fragment of an antibody having specificity for a characterised epitope of hen egg lysozyme (HEL) was produced by expression of heavy variable ($V_H$) and light variable ($V_L$) polypeptides in E.coli and reconstitution in vitro.

A panel of five murine monoclonals, Gloop 1 - 5, have been raised to a well characterised epitope, the loop of hen egg lysozyme (HEL), (M.J. Darsley and A.R. Rees, (1985a)). Genes coding for the heavy and light chains of Gloop 2 (IgG2b, k) have been cloned and the nature of this antibody's interaction with HEL has been analysed by the combined strategy of site-directed mutagenesis (SDM) and a computer model derived from complementarity determining region (CDR) homology in known crystal structures (P. de la Paz, B.J Sutton, M.J. Darsley and A.R. Rees, (1986); S. Roberts and A.R. Rees, (1986); and S. Roberts et al, (1987).

In the manipulation of the cloned Gloop2 heavy and light chain genes two amino-acid codons from the 5′ end of $\gamma$2b were included in the heavy-chain variable-region gene (vhg2); vlg2 consisted of light chain variable region sequences alone. Transcription signals were present in the parent dual-origin vector chosen for expression (pMG939). The parent vector contained the gene for a mammalian growth hormone and was used as a control for temperature-inducible expression and reconstitution of Pep-1 binding activity. The vector was derived from pMG411 (Wright et al., 1986)

Site-directed mutagenesis of the Gloop2 genes was according to the method of Carter et al. (1985), except that for the double mutagenesis (vlg2) the two mutagenic and the selection oligonucleotides were included in equimolar amounts to a total concentration equal to that described.

Oligonucleotides were made on an Applied Biosystems International plc Model 381A DNA synthesiser, and purified as described by Roberts and Rees (1986).

The entire vlg2 sequence was ascertined using the method of Sanger et al., (1977) and that of vhg2 by both dideoxy and Maxam and Gilbert sequencing.

Linker insertion at the 5′ end of vhg2 (in pAT153) used a 17mer and a 13mer oligonucleotide, phosphorylating at only the blunt site. A 100 times molar ratio of oligonucleotides to vector fragment was used. Ligation was as described by Maniatis et al., (1982). The presence of the linker was screened for using radiolabelled 17mer as described by Carter et al., (1984), performing the prehybridisation in sealed bags at ambient temperature overnight. Colonies showing very strong signals were sequenced by the method of Maxam and Gilbert (1977).

Both rFv genes were excised with Bg12 and EcoR1 and after purification from low melt agarose by the method of Maniatis et al., (1982), ligated into the similarly purified Bg12-EcoR1 vector fragment from pMG939. ligation was assessed by restriction analysis.

Gloop2 $V_H$ and $V_L$ polypeptides were directly expressed in severely protease-deficient strains of E. coli, i.e., htpR⁻, lon⁻. These formyl-Methionine derivatives consisted of 116 and 109 amino acids for $V_H$ and $V_L$ respectively. The expression of the Gloop2 polypeptides was analysed by biosynthetic labelling in vivo - (Figure 2).

In vigorously growing E. coli strain 1B373 both polypeptides were produced, but only $V_L$ was present at high levels after 2 minutes. Neither polypeptide accumulated in this strain or in the naturally occuring lon⁻ strains B and B/r, as assessed by Coomassie-stained SDS-PAGE (not shown). Accumulation was detected only in host strains of E. coli deficient in the heat shock response, i.e., htpR- strains (Baker et al., 1984) (Figure 3C,D). A lon⁻ mutation in this background (CAG629) allowed even greater accumulation (Figure 3D), and this host was therefore used as the production strain.

Their small size, and their hydrophobicity, makes the $V_H$ and $V_L$ formyl-methionine derivatives vulnerable to heat-shock proteases, perhaps because they mimic the intracellular signals generated by stress that activate the heat shock system.

La, encoded by lon, is well-known for its scavenging properties particularly in the presence of small or abnormal polypeptides (Waxman and Goldberg, 1982); it is a heat-shock protein under htpR regulation (Goff et al., 1984). htpR⁻ host strains have allowed product accumulation for other small proteins such as the 8.5 kDa complement factor C5a (Mandecki et al., 1986) and the 70 amino-acid somatomedin-C (Buell et al, 1985). The htpR gene encodes a positive regulator of the heat-shock response, a 32-kDa σ factor for core RNA polymerase (Grossman et al., 1984) conferring specificity for heat shock promoters. The continuous presence of this and other factors (Zimarino and Wu, 1987) ensures the rapid response to stimulus, whether temperature or abnormal polypeptides (Burdon, 1986), observed when $V_H$ and $V_L$ were produced following heat-shock induction. The compensating activity of protease Ti (Hwang et al., 1987) may explain the lack of V accumulation in lon⁻ strains.

$V_H$ expression was improved by a longer Shine-Dalgarno sequence. Production of $V_H$ was augmented 2-fold by insertion of a 9 base-pair (bp) Shine-Dalgarno sequence (5'-AAGGAGGTA) into the trp ribosome binding site (vector pPW.HF2). Analysis by in vitro synthetic labelling showed improved expression (Figure 3A) which resulted in increased accumulation of $V_H$ in CAG629 (Figure 3B,C). No significant changes in production levels were noted for $V_L$, although if anything, expression was decreased (Field, 1988)

The longer Shine-Delgarno sequence had no effect on production of the more stable growth hormone, but may prove useful for the production of some smaller polypeptides (e.g., ≤ 14.5 kDa). Alternative SD and leader sequences have previously been used to enhance expression (Greenfield et al., 1986; Sung et al., 1986). Computer analysis of the ability of the message to base-pair with the 16S ribosomal RNA indicated that the longer Shine-Dalgarno may establish more stable binding to the appropriate ribosomal RNA sequence than did the original trp promoter. This is in agreement with other studies demonstrating that the affinity of the ribosome for the messenger RNA is important for efficient translation (Coleman et al., 1985; Jacob et al., 1987). The influence of secondary structure in the ribosome binding site will depend on the gene sequence itself, thus the lack of improvement noted for $V_L$ expression (in vitro expression was decreased) may be explained by the introduction of unfavourable secondary structure into this region.

The influence of the post-initiation AUG sequences on gene expression is becoming well-documented (Looman et al., 1987; Kiltunow et al., 1987). Manipulation of the Shine-Dalgarno to AUG start codon distance is also known to affect synthesis (Fujisawa et al., 1985). However, reduction of the number of base-pairs from 14 to 12, 11 or 10 had no pronounced effect on $V_H$ production from pMG.HF2, although once this distance was reduced to 5 bp, expression was greatly diminished (Field, 1988). Computer studies also indicated the $V_L$ was expressed at high levels due to enhanced messenger stability: a region of internal homology at the 3' end of the messenger RNA was postulated to form stable secondary structure deflecting 3'→5' acting exoribonucleases (Glass, 1982). This internal homology was absent in vhg2 (Field, 1988).

The microvax programs BESTFIT and COMPARE were used for analysis of homology. FOLD was used to assess the secondary structure of the messenger RNA. The sequence for the E. coli 16S ribosomal rRNA was taken from file ecotgix.seg, in the UIC database (University of Wisconsin).

Transformed cell lines were grown at 30°C overnight, in L-broth containing 100 $\mu$g.ml⁻¹ ampicillin and, for CAG629, 10 $\mu$g.ml⁻¹ tetracyclin also. 50mls L-broth containing drugs as appropriate were innoculated at 0.08 $A_{600nm}$ and grown at 30°C, shaking at ca. 200 rpm, to an $A_{600}$ of 0.4, or 1.0 for htpR⁻ strains, transferred to a 45-50°C water bath and flash-induced to 42°C, shaking frequently. Cultures were then grown for up to 6 hours at 37°C, shaking, to allow product accumulation. Other volumes of cultures, e.g., 1 litre or 10mls, were grown, innoculating and inducing at the same absorbance

Both $V_H$ and $V_L$ products were found in the insoluble fraction of host cells, the inclusion bodies (IBs) (Field et al., 1988). The $M_r'$ of $V_H$ and $V_L$ were similar, and just less than 14 kDa. Excessive hydrophobicity may account for this apparently diminished $M_r$, as noted for the Fv polypeptides produced by Hochman et al., (1973).

Fractionation of cell extracts into soluble and insoluble (inclusion body, IB) material was performed using the method of Schoemaker et al., (1985) with the following alterations: cells were lysed in a buffer containing 100mM Tris.HCl pH 7.5, 5mM EDTA and 270 $\mu$g. ml$^{-1}$ lysozyme, and incubated with 0.2 mM PMSF for 5 minutes on ice. Sodium deoxycholate was added as described and incubated on ice; the final incubation, on ice for 5 minutes, was with 0.04mg.ml$^{-1}$ DNase I and 10mM MgCl$_2$.

Reconstitution and partial purification of the rFv and controls was accomplished either following the protocol of Cabilly et al., (1984) with reconstitution from cell lysates, or the IBs were extracted by cell lysis with lysozyme as described above, or else IBs were extracted following French pressure cell lysis (sonication in the case of the growth hormone and host cell controls). At each stage of the manipulation (preceding solubilisation in guanidine), 50 $\mu$l 100mM phenylmethylsulphonyl fluoride (PMSF) and 35 $\mu$l of a cocktail of protease inhibitors (1mM EDTA/10 $\mu$g.ml$^{-1}$ leupeptin/-10 $\mu$g.ml$^{-1}$ pepstatin/200 $\mu$g.ml$^{-1}$ bacitracin/1mM benzamadine-HCl/0.1mM PMSF/20 $\mu$g.ml$^{-1}$ soybean trypsin inhibitor) were added. Pellets were washed twice with 2% Triton-X100 and once with water, dissolved in 7.6M guanidine-HCl, 50mM Tris-HCl pH8, 1mM EDTA, 0.1M $\beta$-mercaptoethanol, and incubated for one hour at 37ºC.

The extracts were diluted to give a potential rFv content of 50 $\mu$g.ml$^{-1}$ in 8M guanidine-HCL/50mM Tris-HCl pH 8/1mM EDTA. $V_H$ and $V_L$ were also reconstituted separately and mixed at 50 $\mu$g.ml$^{-1}$, while growth hormone and host cell control extracts were added to approximately the same host-cell protein content. These were each, separately reconstituted according to the method of Cabilly et al., (1984) except that 1 ml protease inhibitors were added to the final phosphate buffered saline dialysis medium. All reconstitutions were concentrated using Centricon-10 (Amicon), and fractionated by gel filtration using Fast Protein Liquid Chromatography (FPLC) through Superose 12 (Pharmacia).

The process was also scaled-up and purification to homogeneity carried out. 2 litres $V_H$-culture and 0.6 litres $V_L$-culture were lysed in 30mls and 10mls lysosyme lysis buffer containing protease inhibitors (above). Lysed cells were centrifuged in 30ml Corex tubes at 1200g for 10 minutes, then washed three times with 2% Triton-X100 in 100mM Tris-HCl pH 7.5/5mM EDTA, adding protease inhibitors each time. Pellets were resuspended in 20mls and 10mls of 6M guanidine buffer (as above).

Extracts were fractionated on a 300ml (1.5m) Sephacryl S-200 (Pharmacia) column equilibrated in 6M guanidine-HCl/50mM Tris-HCl pH 8.0/5mM EDTA/1mM $\beta$-mercaptoethanol. $V_H$- and $V_L$- containing fractions were pooled separately. A potential rFv concentration of 200 $\mu$g.ml$^{-1}$ was reconstituted as described above, using 30mls per dialysis bag per 625mls urea buffer. Following concentration by ultrafiltration using a YM5 membrane (Amicon) the reconstitutions were further fractionated on the same Sephacryl S-200 column equilibrated in PBS. The rFv fractions were pooled and dialysed into 20mM Tris pH 6.34 and loaded onto a 13ml (20cm) DEAE (DE-52, Whatman) column equilibrated in the same buffer. rFv eluted in the void. For refolding by dilution, guanidine lysates were diluted from 5M to 2M guanidine with PBS pH 7.5, mixed and then further diluted to 0.6M guanidine. Pep1 binding of a control prepared by dialysis as above, was compared.

In order to demonstrate the reconstitution of rFv heteroduplex in partially purified material washed IBs or frozen host cell pellets were solubilised and reconstituted. Binding to Pep-1, a proteolytic fragment of HEL containing the loop antigen (and to which the Gloops were raised), was tested by the competition assay described below, and found only in reconstitutions containing putative rFv, and not in controls consisting of similarly prepared host cells, host cells carrying the parent vector (expressing recombinant growth hormone), or $V_H$ and $V_L$ alone (not shown). The presence of lysozyme in the lysis buffer was demonstrated not to alter Pep1 binding.

These reconstitutions were partially purified by gel filtration through Superose-12 (Figure 4). A Pep-1 binding complex in $V_H$ plus $V_L$ reconstitutions eluted with a retention time consistent with the apparent $M_r$ of a dimer ($\approx$ 21,000). A peak on the $A_{280nm}$ elution profile at this position was unique to putative rFv-containing fractions, and eluted close to the position of growth hormone ($M_r'$ 22,500). Pep-1 binding was also obtained from reconstitutions of $V_H$ or $V_L$ alone, eluting at a retention time consistent with monomers, at $M_r' \approx 12,000$ (in fraction 17). $V_H$ and $V_L$ therefore did not form homodimers, and rFv must be heterodimeric.

The binding assay comprised binding 50 $\mu$l per well of 10 $\mu$g.ml$^{-1}$ affinity purified goat anti-mouse IgG in PBS/0.02% sodium azide or coating buffer (0.05M sodium carbonate/bicarbonate, pH 9.6/0.02% sodium azide) to microtitre plates at 4ºC overnight, or 4 hours at ambient. After washing for 3x 1 minute with PBS/0.05% Tween 20/0.02% sodium azide, the wells were blocked with 0.5% casein in PBS/azide or coating buffer, for 1-2 hours at 37ºC. After washing, Gloop2 at 0.5(-2) $\mu$g.ml$^{-1}$ was added as a second antibody layer in washing buffer, incubating for 4 hours at ambient or overnight at 4°C. The wells were washed and test samples were serially diluted in triplicate (for the partially purified rFv/controls) or quadruplet for pure rFv assays (25 l Superose 12 fractions were tested in duplicate), into 25 $\mu$l PBS/azide. $^{125}$I-labelled Pep 1 in washing buffer was added to a final volume of 50 $\mu$l. 100% values were obtained by adding only labelled Pep 1 to the well; non-specific binding (0%) was obtained by adding 100 $\mu$g.ml$^{-1}$ Gloop2 with the hot Pep 1. After equilibration overnight at 23°C plates were washed and individual wells cut out and counted on an LKB Clinigamma counter. Computer analysis of the binding data was using the program SANCOL (R Ryan, 1988).

Protein concentrations were determined using the BCA assay (Pierce) and purity estimated by BCA and weighing densitometry traces made using a Joyce Mk III C8 double beam recording microdensitometer. Amino-acid analysis was performed using the Pico Tag (TM) system (Waters Associates Inc.)

Following the final step of purification, shown in Figure 5, Gloop2 rFv was subjected to amino-acid analysis (Table 1). Correct amino acid composition and a purity of > 98% was demonstrated. Purity was confirmed by silver-stained SDS-PAGE.

## Table 1

### Amino acid composition of purified rFv

| Amino acid | Theoretical composition | Observed composition (moles amino acid/mole rFv) |
|---|---|---|
| Ala | 16 | 14.9 |
| Arg | 13 | 12.8 |
| Asx | 9 | 9.0 |
| *Cys | 4 | 1.6 |
| Glx | 25 | 22.8 |
| Gly | 19 | 17.5 |
| His | 0 | 0.2 |
| Ile | 9 | 9.4 |
| Leu | 22 | .23.0 |
| +Lys | 12 | 18.2 |
| Met | 4 | 3.2 |
| Phe | 8 | 10.4 |
| Pro | 6 | 5.7 |
| *Ser | 32 | 27.6 |
| *Thr | 22 | 20.3 |
| Trp | 4 | - |
| +Tyr | 13 | 15.2 |
| Val | 7 | 7.1 |
| Total No. | 225 | |

Table 1 : The amino acid composition of purified rFv was obtained by Pico Tag analysis, with amounts obtained in nmoles. Values were normalised to Asx. *Errors are known to occur in Cys, Ser, Thr because of oxidation (underestimated); +Tyr and Lys may be masked by Tris peaks (overestimated). Errors in the range of 2% for the more reliable estimates.

The antigen (Pep-1) binding of rFv was compared with that of Fab'(Gloop2) and the whole MAB (Figure 6). Within the limits of this type of experiment, the $K_d$s were in good agreement, at 21 nM for whole Gloop2, 17nM for Fab'(Gloop2) and 9nM for rFv(Gloop2).

The extinction coefficient, $\epsilon$, was experimentally determined to be 38300 $M^{-1}.cm^{-1}$ ($A_{280} \equiv 26 \mu M$), compared with a theoretical value of 35620, obtained by addition of Trp and Tyr extinction values.

Purified rFv(Gloop2) retaining the binding activity observed in the intact monoclonal antibody may offer a solution to some of the problems encountered in studies of antigen-antibody binding. Studies are hampered by a low rate of production of protein antigen-antibody (Fab') co-crystals. The availability of antigen binding fragments in large amounts will allow physical studies.

Table 2

| Purification step | | $Fv/V_H/V_L$ (mg) | Yield % | Purity % |
|---|---|---|---|---|
| Cells | $V_H$ (2litres) | 35.5 | 100 | 2 |
| | $V_L$ (1/3.5 Litres) | 40.0 | 100 | 7 |
| Inclusion bodies | | >26-30 | >90 | 7-12 |
| S-200/guanidine | | 25-40 | 75-100 | 13-30 |
| Reconstitution | | 4.8* | 6 | 31 |
| S-200/PBS | | 3.8 | 5 | 42 |
| DEAE | | 1.6 | 1.9 | >98 |

Table 2: yields of rFv or its components obtained throughout the purification protocol described. During reconstitution, BSA was added as a carrier.

*This figure represents loss of protein during dialysis: 80% losses were recorded at this stage. Reconstitution of $V_H$ and $V_L$ into active Fv is in itself an efficient process.

Expression levels of $V_H$ and $V_L$ were estimated at 2% and 7% of total bacterial protein, respectively. Using Cabilly's protocol for reconstitution, and other fractionation methods as described, 1 litre of shake-flask culture expressing $V_H$ and 0.3 litre $V_L$ culture yielded 0.7 mgs 98% pure rFv, representing 1.9% of the starting material (see Table 2). This low yield was due mainly to protein losses during reconstitution, where more than 80% of V polypeptides were lost. This problem was later alleviated by reconstitution involving a simple dilution of V polypeptides from guanidine into phosphate-buffered saline pH 7.5.

Hochman et al., (1973) have demonstrated the reconstitution of variable domains to be a very efficient process, with 87% recovery of the original binding activity. This compared favourably with the 17% recovery of Fab' activity from fully denatured polypeptides (Haber, 1964). The presence of the second domain militated against efficient refolding due to interdomain interactions (Tsunenaga et al., 1987).

In addition, cultures may be industrially fermented to up to 40 times the cell density obtainable at bench-level (harvesting $OD_{600nm}$ was ≈ 1.5) allowing us to predict that tens of milligrams of purified rFv may be produced from 1 litre each of cells expressing $V_H$ and $V_L$.

In vitro translation employing the method supplied with the prokaryotic translation kit from Amersham International was followed and the $^{125}$I-Met labelled products analysed by SDS-PAGE and autoradiography. Molecular weight protein markers were either $^{14}$C-labelled (Amersham) or prestained (Gibco-BRL).

In Western blotting experiments proteins were transferred from 10-15% PA gels at 60V for 3 hours or at 30V overnight, and the filter probed using the method of Burnette (1981), with the following alterations; the blot sandwich was made from 2 pieces of whatman 3MM enclosing the gel and nitrocellulose (Schleicher and Schuell) of 0.1 μm pore size. The electrode solution contained 25mM Tris base, 192mM glycine and 20% methanol. Blocking was with 0.9% saline, 20mM Tris pH 7.6, 0.05% Triton X-100 and 0.5% casein hammarsten (BDH), with four changes over 1 hour at ambient. The anti-$V_H$ or -$V_L$ sera (below) were applied by spreading in 1ml blocking buffer, and incubated in a humidity chamber for 1 hour at ambient. Washing was with four changes of buffer without casein, and 2-4 μCi $^{125}$I-labelled Protein A added in the same manner as the antisera. After incubating at ambient for 1 hour, the filter was washed in a high salt wash buffer containing 1M NaCl, 20mM Tris pH 7.6 and 0.4% N-lauroyl sarcosine four times at ambient, air dried and autoradiographed.

To prepare rabbit antisera chloramphenicol acetyl transferase (CAT) fused to $V_H$ or $V_L$ at its C-terminus here produced in E. coli. The vector pCT202 was used to construct the vectors direct in expression of CAT-$V_H$ and CAT-$V_L$. Insoluble material was prepared for both recombinants, which were electroeluted following preparative SDS-PAGE (with or without Coomassie staining) using a Biotrap system (Schleicher and Schuell). After dialysis into PBS the protein concentration was estimated and injected using the following

schedule: ≈ 60 μg in complete Freunds adjuvant in 1ml intradermally into the back and scruff; after 16 days, 130 μg in incomplete Freunds in 0.8ml intramuscularly into the haunch; after a further 38 days, 50 μg in PBS was injected into haunch and scruff, and the next day 200 μg in PBS intravenously into the ear vein. Bleeding was also from the ear vein, one week later.

The route for direct polypeptide expression in E. coli, using an inducible vector in conjunction with heat-shock protease deficient host strains was useful for production of another, unrelated rFv.

Example 2

The Fv fragment of an antibody having specificity for a carbohydrate epitope of a breast carcinoma antigen (Colcher et al., 1981) was produced by expression of heavy variable ($V_H$) and light variable ($V_L$) polypeptides in E. coli and reconstituted in vitro in the same manner as for the Gloop2 Fv.

This murine monoclonal antibody will have potential application in tumour therapy. To reduce its immunogenicity in the human system, a chimera was constructed (Whittle et al., 1987). The variable region was placed onto human constant regions by genetic engineering. The retension of binding activity demonstrated the independence of the binding of the variable region (or Fv).

$V_H$ and $V_L$ genes were constructed from the previously cloned complete (but unrearranged - see Whittle et al., op. cit.) heavy and light chain genes by insertion of an ATG (encoding formyl-Methionine) start and a stop codon. A 3′ Bg12 and a 5′ EcoRI site were also engineered, such that the entire Fv sequence was contained in two genes and bounded by restriction sites compatible with the expression vector used.

5′ end manipulation was by insertion of linkers, as described for Gloop2 $V_H$ gene construction. The linker region sequences were verified by dideoxy sequencing of the double-stranded plasmids, after insertion into the vector, pMG939.

The growth hormone gene of the vector described in the previous example, pMG939, was removed, and the $V_H$ or$V_L$ genes for B72.3 expression inserted, forming the plasmids pMG.ROH4/9 and PMG.ROL5/10.

The dual origin vector above was used to produce both heavy and light polypeptide chains (120 and 109 amino acids, respectively) in the protease deficient strain E. coli CAG629 ($htpR^-$ and $lon^-$) (Figure 8). Both polypeptides appeared in the insoluble fraction of host cells (Figure 9).

Concluding Remarks

The small size of rFvs should simplify studies of antibody combining sites by physical methods. For X-ray crystallography, the switch site angle (between variable and constant domains) need not be determined, reducing computation times, and the similarity of tertiary structure between rFvs will allow rapid assessment of data from new rFv crystals. Furthermore, not all Fabs may be induced to crystallise (Davies and Metzger, 1983), and it is possible that rFvs, lacking the flexible elbow bend, will crystallise more easily. The ability to produce $V_H$ and $V_L$ separately will allow their complete assignment by NMR spectroscopy, and assessment of the interdomain interaction.

E. coli will not glycosylate its products. Although the variable domains of immunoglobulins are non-stoichiometrically glycosylated in vivo, the carbohydrate is not usually involved in antigen-binding (Taniguchi et al., 1985) and specifically, absence of carbohydrate is found not to effect IgG2b function (Nose and Wigzell, 1983).

The conserved nature of variable domain pairing (Chothia et al., 1985) suggests that the methods described here for reconstitution of rFv binding from chaotropic buffer will also apply to any rFv. The methods described here will be employed in antigen-binding studies. A potentially high yield, cytoplasmic route for V polypeptide expression may encourage the development of immunotoxin constructs, or chimeric therapeutic agents with low immunogenciity.

References

Baker, T.A., Grossman, A.D. and Gross, C.A. (1984) Proc. Natl. Acad. Sci. USA, 81, 6779-6783.
Boss, M.A. Kenten, J.H., C.R. & Emtage, S.J. (1984) Nucleic Ac. Res. 12, 3791-3806.
Buell, G., Schulz, M-F Selzer, G., Chollet, A., Movva, N.R., Semon, D., Escanez, S., Kawashima, E. (1985) Nucleic Ac. Res. 13, 1923-1938. Burdon, R.H. (1981) Biochem. J. 240, 313-324.
Burnette, W.N. (1981) Anal. Biochem. 112, 195-203.
Cabilly, S, Riggs, A.D., Pande, H., Shively, J.E., Holmes, W.E., Rey, M., Perry, L.J., Wetzel R. & Heyneker, H.L. (1984) Proc. Natl. Acad. Sci. USA 81, 3273-3277.

Carter, P., Bedouelle, H. & Winter, G. (1985) Nucleic Ac. Res. 13, 4431-4442.

Carter, P.J., Winter, G., Wilkinson, A.J. & Fersht. (1984) Cell 38, 835-840.

Chothia, C., Novotny, J., Bruccoleri, R. & Karplus, M. (1985) J. Mol. Biol. 186, 651-663.

Colcher, D., Hand, P.H., Nuti, M., Schlom, J. (1981) Proc. Natl. Acad. Sci. USA 78, 3199-3203.

Coleman, J., Inouye, M. & Nakamura, K. (1985) J. Mol. Biol. 181, 139-143.

Darsley, M.J. & Rees, A.R. (1985a) EMBO J. 4, 383-392.

Darsley, M.J. & Rees, A.R. (1985b) EMBO J. 4, 393-398.

Davies, D.R. & Metzger, H. (1983) Ann. Rev. Immunol. 1, 87-118.

de la paz, p., Sutton, B.J., Darsley, M.J. & Rees, A.R. (1986) EMBO J. 5, 415-425.

Field, H (1988) D.Phil thesis, Oxford University, U.K.

Field, H., Yarranton, G.T. Rees, A.R. (1988) In "Vaccines '88'", Cold Spring Harbor Publications, Pages 29-34.

Fujisawa, Y., Ito, Y., Ikeyama, S. & Kikuchi, M. (1985) gene 40, 23-29.

Glass, R.E. (1982) gene Function. E. coli and its heritable elements. Croom helm, London, pp. 58-70.

Goff, S.A., Casson, L.P. & Goldberg, A.L. (1984) proc. Natl. Acad. Sci. USA 81, 6647-6651.

Greenfield, L., Dovey, H.F., Lawyer, F.C. & Gelfand, D.H. (1986) Bio/Technology 4, 1006-1011.

Grossman, A.D., Erickson, J.W. Gross, C.A (1984) Cell 38, 383-390.

Haber, E. (1964) Proc. Natl. Acad. Sci. USA 52, 1099-1106 Hendershot, L., Bole, D. and Kearney, J.F. (1987) Immunology Today 8, 111-114.

Hochman, J., Inbar, D. & Givol, D. (1973) Biochemistry 12, 1130-1135

Hwang, B.J., Park, W.J., Chung, C.H. & Goldberg, A.L. (1987) Proc. Natl. Acad. Sci. USA 84, 5550-5554.

Jacob, W.F., Santer, M. & Dahlberg, A.E. (1987) Proc. Natl. Acad. Sci. USA 84, 4757-4761.

Kenten J., Helm, B., Ishizaka, T., Cattini, P. & Gould, H. (1984) Proc. Natl. Acad. Sci. USA 81, 2955-2959.

Klausner, A. (1986) Bio/Technology 4, 1041-1043.

Koltuno, A.M. Gregg, K. & Rogers, G.E. (1987) Nucleic Ac. Res. 15, 7795-7808.

Looman, A.C., Bodlaender, J., Cornstock, L.J., Eaton, D., Jhurani, P., de Boer, H.A. & van Knippenberg, P.H. (1987) EMBO J. 6, 2489-2492.

Mandecki, W., Powell, B.S., Mollison, K.W., Carter, G.W. & Fox, J.L. (1986) Gene 43, 131-138.

Maniatis, T., Fritsch, E.F. & Sambrook, J. (1982) Molecular Cloning: A Laboratory Manual. Cold Spring Harbor Laboratory, Cold Spring Harbor, New York.

Maxam, A.M. & Gilbert, W. (1987) Proc. Natl. Acad. Sci USA 74, 560-654.

Nose, M. & Wigzell, h. (1983) Proc. Natl. Acad. Sci. USA 80, 6632-6636.

Roberts, S., Cheetham, J.C. & Rees, A.R. (1987) Nature (Lond.) 328, 731-734.

Roberts, S. & Rees, A.R. (1986) Protein Engineering 1, 59-65.

Ryan, R. (1988) D. Phil thesis, Oxford University, U.K.

Sanger, F., Nicklen, S. & Coulson, A.R. (1987) Proc. Natl. Acad. Sci. USA 74, 5463-5467.

Schoemaker, J.M., Brasnett, A.H. & Marston, F.A.O. (1985) EMBO J. 4 775-780.

Sen, J. and Beychok, S. (1986) Proteins: structure, function & genetics 1, 256-262.

Sharon, J. & Givol, D. (1976) Biochemistry 15, 1591-1594.

Sung, W.L., Yao, F-L., Zahab, D.M. & Narang, S.A. (1986) Proc. Natl. Acad. Sci. USA 83, 561-565.

Taniguchi, T., Mizuochi, T., Beale, M., Dwek, R.A., Rademacher, T.W. and Kobata, A. (1985) Biochemistry 24, 5551-5557.

Tsunenaga, M., Goto, Y., Kawata, Y., Hamaguchi, K. (1987) Biochemistry 26, 6044-6051.

Verhoeyen, M. and Riechmann, L. (1988) Bioessays 8, 74-78

Waxman, L. & Goldberg, A.L. (1982) Proc. Natl. Acad. Sci USA 79, 4883-4887.

Whittle, N., Adair, J., Lloyd, C., Jenkins, L., Devine, J., Schlom, J., Raubitschek, A., Colcher, D., Bodmer, M (1987) Protein Engineering 1, 499-505.

Wright, E.M., Humphreys, G.O. & Yarranton G.T. (1986) gene 49, 311-321. Zimarino, V. & Wu, C. (1987) Nature (Lond.) 327, 727-730.

**Claims**

1. A process for the production of an immunologically functional Fv antibody fragment comprising direct expression of heavy and light chain peptides comprising complementary variable domains only in a bacterial host strain which is protease-deficient.

2. The process of claim 1 in which the heavy and light chain polypeptides are expressed by means of an inducible expression system in which copy number is inducible.

3. The process of claim 1 or claim 2, wherein the protease-deficient bacterial host strain is <u>E. coli</u>.

4. The process of claim 3, wherein the protease-deficient bacterial host strain is a lon⁻ strain.

5. The process of claim 3, wherein the protease-deficient bacterial host strain is a hptR⁻ strain.

6. The process of any preceding claim, wherein the protease-deficient bacterial host strain is multiply protease-deficient.

7. A copy number inducible bacterial expression vector for the production of an immunologically functional Fv antibody fragment in a protease-deficient bacterial strain containing a DNA sequence coding for a polypeptide comprising a variable domain only wherein the DNA sequence is located within the vector such that it is expressed as a direct expression product.

8. A copy number inducible bacterial expression vector for use in a protease-deficient bacterial strain containing DNA sequences coding for heavy and light chain polypeptides comprising variable domains only wherein the DNA sequences are located within the vector such that they are expressed as direct expression products.

9. A protease-deficient bacterial host cell transformed with an expression vector according to claim 7 or claim 8.

**Patentansprüche**

1. Ein Verfahren zur Herstellung eines immunologisch funktionellen Fv-Antikörperfragmentes, bei welchem Verfahren direkte Expression von Schwere-Ketten und Leichte-Ketten-Peptiden, die nur komplementäre variable Domänen enthalten, in einem bakteriellen Wirtsstamm, der Protease-defizient ist, erfolgt.

2. Das Verfahren nach Anspruch 1, bei welchem die Schwere-Ketten- und Leichte-Ketten-Polypeptide mit Hilfe eines induzierbaren Expressionssystems, in dem die Kopienzahl induzierbar ist, exprimiert werden.

3. Das Verfahren nach Anspruch 1 oder Anspruch 2, bei welchem der Protease-defiziente bakterielle Wirtsstamm <u>E. coli</u> ist.

4. Das Verfahren nach Anspruch 3, bei welchem der Protease-defiziente bakterielle Wirtsstamm ein lon⁻-Stamm ist.

5. Das Verfahren nach Anspruch 3, bei welchem der Protease-defiziente bakterielle Wirtsstamm ein hpt⁻-Stamm ist.

6. Das Verfahren nach einem der vorhergehenden Ansprüche, bei welchem der Protease-defiziente bakterielle Wirtsstamm mehrfach Protease-defizient ist.

7. Ein bakterieller Expressionsvektor, dessen Kopienzahl induzierbar ist, für die Herstellung eines immunologisch funktionellen Fv-Antikörperfragmentes in einem Protease-defizienten Bakterienstamm, der eine für ein Polypeptid mit nur variabler Domäne codierende DNA-Sequenz enthält, wobei die DNA-Sequenz innerhalb des Vektors lokalisiert ist, sodaß sie als ein direktes Expressionsprodukt exprimiert wird.

8. Ein bakterieller Expressionsvektor, dessen Kopienzahl induzierbar ist, zur Verwendung in einem Protease-defizienten bakteriellen Stamm, der DNA-Sequenzen enthält, die für Schwere-Ketten- und Leichte-Ketten-Polypeptide mit nur variablen Domänen codieren, bei welchem die DNA-Sequenzen innerhalb des Vektors lokalisiert sind, sodaß sie als direkte Expressionsprodukte exprimiert werden.

9. Eine Protease-defiziente bakterielle Wirtzelle, die mit einem Expressionsvektor nach Anspruch 7 oder Anspruch 8 transformiert ist.

**Revendications**

1. Procédé de production d'un fragment d'anticorps Fv à fonction immunologique, comprenant l'expression directe de peptides de chaîne lourde et de chaîne légère comprenant des domaines variables complémentaires seulement dans une souche d'hôte bactérienne qui est déficiente en protéase.

2. Procédé selon la revendication 1, dans lequel les polypeptides de chaîne lourde et de chaîne légère sont exprimés au moyen d'un système d'expression inductible dans lequel le nombre de copies est inductible.

3. Procédé selon la revendication 1 ou la revendication 2, dans lequel la souche d'hôte bactérienne déficiente en protéase est E. coli.

4. Procédé selon la revendication 3, dans lequel la souche d'hôte bactérienne déficiente en protéase est une souche lon$^-$.

5. Procédé selon la revendication 3, dans lequel la souche d'hôte bactérienne déficiente en protéase est une souche hptR$^-$.

6. Procédé selon l'une quelconque des revendications précédentes, dans lequel la souche d'hôte bactérienne déficiente en protéase présente une déficience multiple en protéase.

7. Vecteur d'expression bactérien à nombre de copies inductible pour la production d'un fragment d'anticorps Fv à fonction immunologique dans une souche bactérienne déficiente en protéase contenant une séquence d'ADN codant pour un polypeptide comprenant un domaine variable seulement, dans lequel la séquence d'ADN est située au sein du vecteur, de sorte qu'elle est exprimée comme produit d'expression directe.

8. Vecteur d'expression bactérien à nombre de copies inductible à utiliser dans une souche bactérienne déficiente en protéase contenant des séquences d'ADN codant pour des polypeptides de chaîne lourde et de chaîne légère comprenant des domaines variables seulement, dans lequel les séquences d'ADN sont situées au sein du vecteur, de sorte qu'elles sont exprimées comme produits d'expression directe.

9. Cellule hôte bactérienne déficiente en protéase transformée par un vecteur d'expression selon la revendication 7 ou la revendication 8.

FIG. 1

FIG. 2

FIG. 3

FIG. 4

FIG. 6

FIG. 5

FIG. 7

NATIVE PAGE

REDUCING SDS-PAGE

EP 0 393 045 B1

# FIG. 8

B72·3   V_H(H4)   V_H(H9)   V_L (L5)   V_L (L10)

0  2  4  6  15   0  2  4  6, 15   0  2  4  6  15   0  2  6  15 hours

V_H

←V_L

EP 0 393 045 B1

## FIG. 9

µgBSA  2  5  10  20    $V_H$  B72·3  $V_L$    $V_H$  Gloop 2  $V_L$

EP 0 393 045 B1

# FIG. 10

Fractionation of hv and lv into the insoluble phase

SDS-PAGE